# EUROPÄISCHE PATENTSCHRIFT

(11) **EP 0 483 104 B1**
(45) Veröffentlichungstag und Bekanntmachung des Hinweises auf die Patenterteilung: **11.03.1998**
(21) Anmeldenummer: 91890256.0
(22) Anmeldetag: 25.10.1991
(51) Int. Cl.: A61L 2/12, A61L 2/26, A61L 11/00

(54) **Anlage zum Erhitzen, Desinfizieren oder Sterilisieren von Gütern**
Apparatus for heating, disinfection or sterilization of goods
Installation pour le chauffage, la désinfection ou la stérilisation de produits

(30) Priorität: 25.10.1990 AT 2164/90; 07.12.1990 AT 2487/90; 29.04.1991 AT 894/91; 26.06.1991 AT 1282/91
(43) Veröffentlichungstag der Anmeldung: 29.04.1992
(73) Patentinhaber: Katschnig, Helmut, Dr., A-8750 Judenburg Steiermark (AT)
(72) Erfinder: Katschnig, Helmut, Dr., A-8750 Judenburg, Steiermark (AT); Gagea, Leonard, Dipl.-Ing., A-8750 Judenburg (AT)
(74) Vertreter: Itze, Peter, Dipl.-Ing.

(56) Entgegenhaltungen:
- EP-A- 0 410 306
- DE-C- 3 505 570

## Beschreibung

Die Erfindung bezieht sich auf eine Anlage zum Erhitzen, Desinfizieren oder Sterilisieren von Gütern, in welcher das zu behandelnde Gut mittels Mikrowellen erhitzt wird, wobei in die das zu behandelnde Gut enthaltende Behandlungskammer Flüssigkeit, z.B. Wasser, welche gegebenenfalls mit Zusätzen, z.B. Desinfektionsmittel und/oder Duftstoffen, versetzt ist, einbringbar ist.

Bei einer bekannten Ausbildung dieser Art (s. DE-OS 33 17 300) ist im Innenraum der Behandlungskammer eine von einein Außenanschluß unabhängige Flüssigkeitsquelle, beispielsweise ein Beutel, der mit Wasser, gegebenenfalls zusätzlich mit Desinfektionsmittel und/oder Duftstoffen, befüllt ist, vorgesehen. Bei dieser bekannten Ausbildung wird also eine vorgegebene Wassermenge in die Behandlungskammer vor Einschalten derselben eingebracht, unabhängig davon, welches Gut sich in der Behandlungskammer befindet.

Dies kann dazu führen, daß im Falle einer zu großen Wassermenge sehr viel Energie unnötig einzubringen ist, uni das zu behandelnde Gut entsprechend zu erhitzen, zu desinfizieren oder zu sterilisieren. Überdies bleibt dann zu viel Flüssigkeit als Rest übrig, was dazu führt, daß erhöhte Flüssigkeitsmengen gezielt zu entsorgen sind. Schließlich würde eine zu große Wassermenge den Bearbeitungszyklus auch ungebührlich verlängern, womit eine länger dauernde Blockierung des Gerätes gegeben ist. Eine zu geringe Wasserdosierung hingegen würde dazu führen, daß bei trockenem Müll oder sonstigem zu behandelnden Gut es zu einer Selbstentzündung kommen könnte, was dann zu einem Brand des Gerätes führt.

Gemäß der EP-A2-0 410 306 ist eine Anlage zur Sterilisation von Müll, insbesondere Krankenhausmüll, bekannt, bei welcher im Behälter gefüllter Müll durch eine Behandlungskammer geführt wird, wobei durch Injektionseinrichtungen dem Müll Wasser zugesetzt wird. Auch bei dieser Anlage erfolgt die Wasserzugabe ohne vorherige Bestimmung der benötigten Wassermenge, wodurch die vorstehend geschilderten Probleme auftreten können.

Erfindungsgemäß werden die angeführten Nachteile dadurch vermieden, daß die Flüssigkeit in Abhängigkeit vom Wassergehalt des zu behandelnden Gutes direkt diesem zusetzbar ist. Dadurch wird erreicht, daß je nach aktuellem Flüssigkeitsgehalt des zu behandelnden Gutes die nötige Flüssigkeitsmenge zugesetzt ist, sodaß unter allen Umständen der für eine zuverlässige Behandlung des Gutes ausreichende Flüssigkeitszusatz erfolgt.

Vorteilhafterweise kann für die Ermittlung des Wassergehaltes des zu behandelnden Gutes eine auf das Gewicht desselben ansprechende Meßeinrichtung vorgesehen sein. In diesem Fall wird von der Annahme ausgegangen, daß das Gewicht des zu behandelnden Gutes im wesentlichen durch den Flüssigkeitsgehalt desselben bestimmt ist. Dies trifft bei den üblichen zu desinfizierenden/sterilisierenden Abfällen von Krankenhäusern od.dgl. in der Regel zu, wobei der Ablauf auch dahingehend gesteuert werden könnte, daß die Meßeinrichtung eine Voreinstellmöglichkeit aufweist, mittels welcher die Art des zu behandelnden Gutes eingestellt werden kann.

Die Meßeinrichtung kann dabei als Wägezelle ausgebildet sein, welche mit einer Steuereinrichtung für die Flüssigkeitszugabe verbunden ist. Damit ist eine automatische Flüssigkeitszugabe direkt in Abhängigkeit von dem Gewicht des zu behandelnden Gutes ermöglicht.

Es kann allerdings das zu behandelnde Gut auch dadurch berücksichtigt werden, daß die Steuereinrichtung der Flüssigkeitszugabe anhand eines den tatsächlichen Flüssigkeitsgehalt berücksichtigenden Algorithmus steuerbar ist. Damit ist es ermöglicht, die Flüssigkeitszugabe optimal an das zu behandelnde Gut anzupassen, wobei in einer etwaigen Steuerung für unterschiedliche zu behandelnde Güter auch unterschiedliche Algorithmen eingegeben sein können.

Um störenden Einflüssen der Mikrowellenstrahlung auszuweichen, kann die Wägezelle außerhalb des Resonanzraumes der Mikrowellen angeordnet sein, wobei als Auflager für die den Behälter für das zu behandelnde Gut bildende tragende Platte bzw. Wandung dient.

Schließlich kann die Flüssigkeitszugabeeinrichtung zur Zuführung der Flüssigkeit in das zu behandelnde Gut innerhalb der Behandlungskammer angeordnet sein, sodaß dann, wenn das Gewicht des zu behandelnden Gutes abgenommen wird, sobald es in die Behandlungskammer eingebracht ist, eine Wasserzugabe innerhalb des gesamtes Gerätes erfolgen kann, ohne daß extern irgendwelche Manipulationen vorgenommen werden müssen.

Um in einem Mikrowellenheizgerät entsprechende physikalische und/oder chemische Parameter erfassen zu können, kann die aus mikrowellenundurchlässigem Material gefertigte Zugabeeinrichtung in eine Öffnung der Behandlungskammer einsetzbar sein, wobei in dem in das Innere der Behandlungskammer ragenden Kopf der Zugabeeinrichtung Sensoren für die Erfassung physikalischer und/oder chemischer Parameter angeordnet sind. Dadurch sind die gegen Hochfrequenzfelder empfindlichen Sensoren innerhalb eines abgeschirmten Bereiches angeordnet, stehen jedoch mit dem Inneren der Behandlungskammer insofern direkt in Verbindung, als ja die Zu-gabeeinrichtung in eine Öffnung der Behandlungskammer hineinragt. Es können damit innerhalb dieses Kopfes die entsprechenden Sensoren in das Innere der Kammer eingebracht werden, wodurch sie dann die entsprechenden gewünschten physikalischen und/oder chemischen Parameter direkt an Ort und Stelle exakt messen können.

Vorteilhafterweise ist der Kopf selbst als Temperaturfühler ausgebildet, d.h. daß die Wandung des Kopfes in gewissen Bereichen als Temperaturgeber für den entsprechenden Sensor wirkt. Es könnten natürlich an der Sensorenwandung auch Druckmeßeinrichtungen, um den Innendruck der Kammer zu wählen, und auch andere Anordnungen angebracht sein, z.B. auch pH-Elektroden, Sauerstoffelektroden u.dgl., um - falls dies erforderlich ist - auch die chemischen Zustände innerhalb des Behälters erfassen zu können. Weiters kann der Kopf der Zugabeeinrichtung als Druckfühler ausgebildet sein, wobei vorzugsweise die Wandung bzw. Bereiche davon als Druckmembrane ausgebildet ist, sodaß bei geeignetem Behältnis auch eine Druckbehandlung des Gutes gesteuert vorgenommen werden kann. Bei nicht druckfestem Behältnis kann bei Auftreten von Überdruck ein Druckregelventil gesteuert betätigt werden.

Zum Aufsetzen der Wassereinbringeinrichtung kann der Kopf der Zugabeeinrichtung nach Schließen der Behandlungskammer auf den Behälter absenkbar sein, welcher in seinem Deckel mit einer vom Kopf der Zugabeeinrichtung abschließbaren Öffnung versehen ist. Dadurch wird zusätzlich zu dem gewünschten Aufsetzen auch der Behälter direkt abgeschlossen.

Um ein Durchmischen des Behälterinneren mit dem eingebrachten Wasser zu erreichen, kann zur Auflage des Behälters ein Drehteller vorgesehen sein, wobei an der Außenseite des Bodens des Behälters Mitnahmeeinrichtungen für das Zusammenwirken mit dem Drehteller angeordnet sind, wobei verhindert ist, daß sich bei einem Wechsel der Drehrichtung des Drehtellers letzterer in bezug auf den Behälter verdreht. Dabei können an der Außenseite des Bodens des Behälters zusätzlich oder anstelle der Mitnahmeeinrichtungen Halteeinrichtungen gegen ein Abgleiten des Behälters vom Drehteller vorgesehen sein.

Falls unmittelbar im Behälter das zu behandelnde Gut gemischt werden soll, können an der Innenseite der Wandung des Behälters nach innen vorspringende, vorzugsweise mit abgerundeten Enden und/oder Kanten versehene Mischleisten od.dgl. angebracht sein. Die Mischleisten können schraubenlinienförmig verlaufen, wobei die Mischleisten durchgehend oder als unterbrochene Abschnitte ausgebildet sind, was zusätzlich zu normaler Turbulenzmischung auch eine Förderung nach oben oder nach unten des im Randbereich befindlichen flüssigen oder breiigen Füllgutes ergibt. Unterbrochene Abschnitte ermöglichen zudem ein besonders leichtes Entleeren des Behälters, da das Füllgut zwischen den einzelnen Mischleistenabschnitten leicht abfließen kann. Die Mischleisten können alle gleichsinnig oder, vorzugsweise abwechselnd, gegenläufig angeordnet sein, wodurch durch die letztgenannte Anordnung eine gleiche Wirkung bei Drehung des Behälters in unterschiedliche Drehrichtungen erreicht wird.

In der Zeichnung sind Ausführungsbeispiele des Erfindungsgegenstandes dargestellt.

Fig. 1 zeigt schematisch den Aufbau der erfindungsgemäßen Anlage.

Fig. 2 ist eine Detaildarstellung des Kopfes der Zugabeeinrichtung.

Fig. 3 gibt ein Ablaufdiagramm für die Ermittlung und die Zugabe der erforderlichen Flüssigkeitsmenge wieder.

Fig. 4 veranschaulicht eine beispielhafte Ablaufkurve eines Algorithmus für die Behandlung von zu desinfizierendem Abfall.

Fig. 5 gibt in Form eines Fließdiagramms die Ermittlung der zuzugebenden Wassermenge anhand des zugehörigen Algorithmus wieder.

Fig. 6 zeigt einen Vertikalschnitt durch den erfindungsgemäßen Behälter bei eingesetztem Deckel.

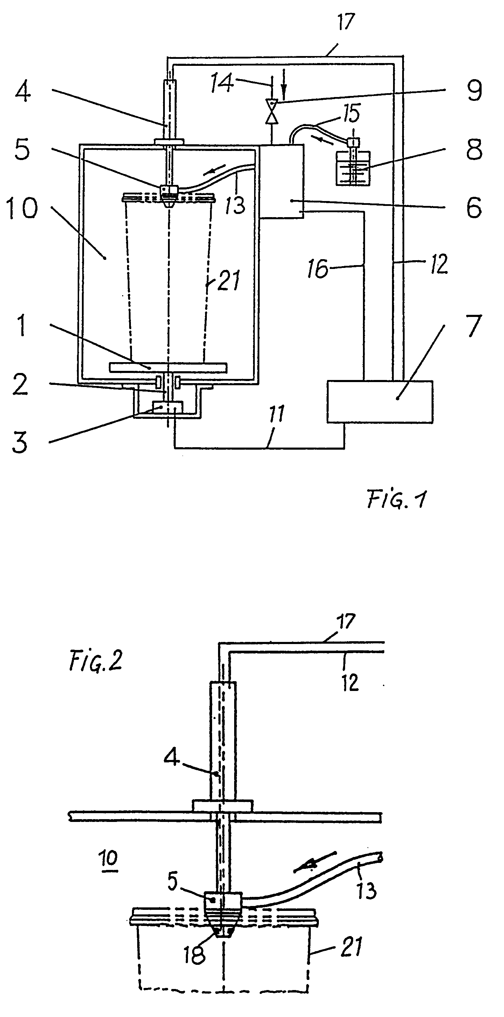

Bei der in Fig. 1 dargestellten Behandlungseinrichtung ist mit 1 der Wägeteller bezeichnet, der zur Abstützung eines Behältnisses 21 für zu behandelndes Gut dient. Der Wägeteller 1 ist auf einer Wägeachse 2 angebracht, welche sich auf einer Wägezelle 3 abstützt. Von dieser Wägezelle 3 führt eine Leitung 11 zu einer Mikroprozessorsteuerung 7, welche über eine Leitung 12 zu einem Stellantrieb 4 führt, über welchen eine Wassereinbringeinrichtung 5 auf das Behältnis aufsetzbar bzw. in dieses von oben her einführbar ist. Diese Wassereinbringeinrichtung 5 ist mit einer Zuführleitung 13 versehen, die zu einer Mischeinrichtung 6 führt, in welche eine Wasserzubringeinrichtung 14 und eine Leitung 15 führt, welch letztere zu einem Behältnis 8 für eine Zusatzlösung, z.B. ein Desinfektions- und/oder Duftmittel führt. In die Leitung 15 ist ein Magnetventil 9 eingeschaltet, mittels welchem die Wasserzufuhr zur Mischeinrichtung 6 steuerbar ist. Die Mischeinrichtung 6 ist über eine Leitung 16 mit der Mikroprozessorsteuerung 7 verbunden. Mit 10 ist die Behandlungskammer bezeichnet, in welche in nicht dargestellter Weise drei Mikrowellengeneratoren gleichzeitig einstrahlen (s. dazu EP-A1-0 287 549). Für die Verwendung als Sterilisator bzw. Desinfektor wird dabei eine Anordnung der Mikrowellengeneratoren vorgesehen werden, die sicherstellt, daß die gesamte Behandlungskammer gleichförmig, also unter Vermeidung von kalten Stellen und Interferenzen, von den Mikrowellen bestrahlt ist, sodaß eine zuverlässige Erreichung aller Stellen der Behandlungskammer und insbesondere des in die Behandlungskammer 10 eingesetzten Gefäßes erreicht ist.

Der Wägeteller 1 kann in nicht dargestellter Weise auf der Wägezelle 3 drehbar gelagert sein, wobei ein nicht dargestellter Schwenkantrieb vorgesehen sein kann, mittels welchem der Wägeteller 1 in eine hin- und hergehende Drehbewegung versetzt werden kann, was z.B. für flüssige Abwässer oder Küchenabfälle notwendig ist, um diese während der Behandlung mit oder ohne der eingebrachten Zusatzlösung gut zu durchmischen und gleichmäßig zu erwärmen.

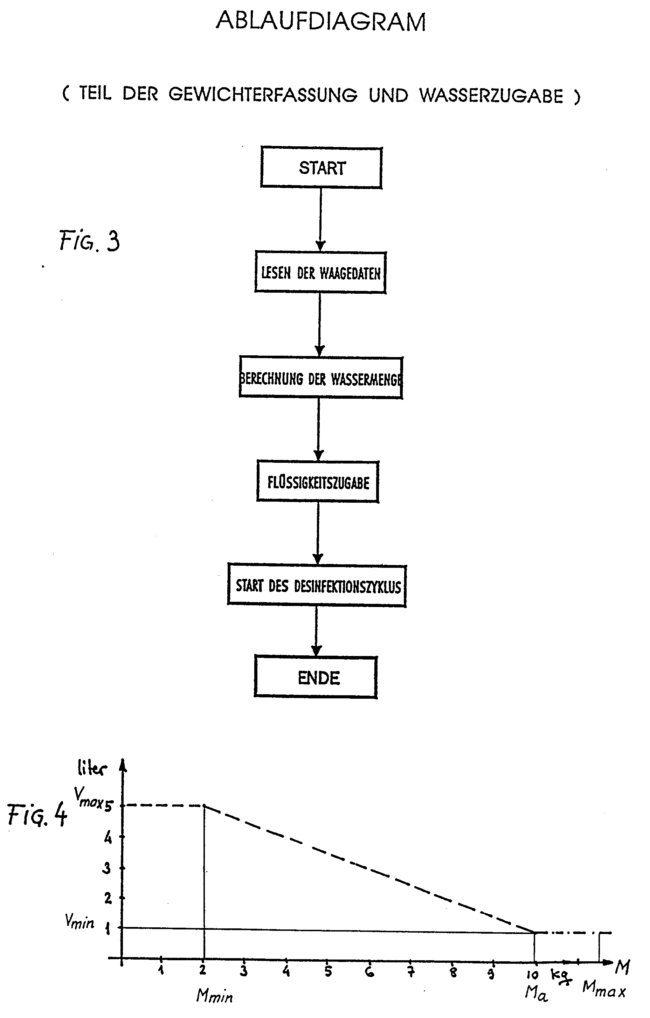

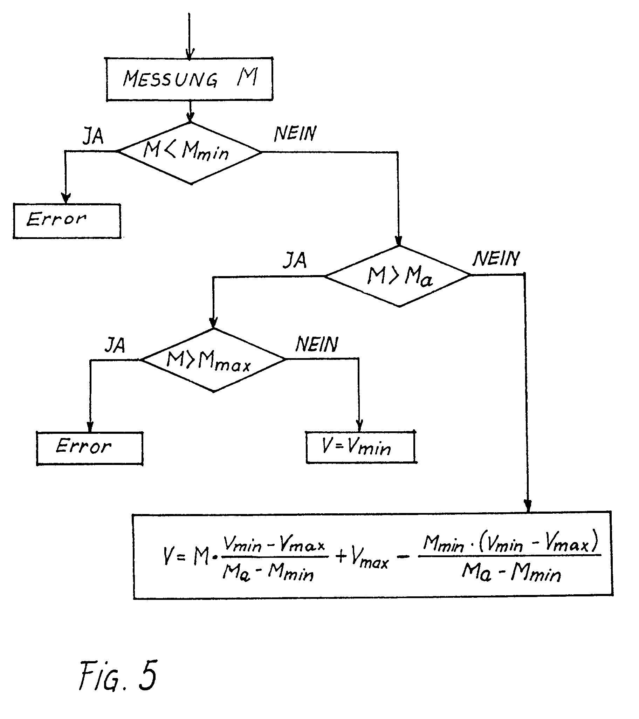

Bei Inbetriebnahme der Anlage zum Erhitzen, Desinfizieren oder Sterilisieren von Gütern wird zunächst das Behältnis 21 mit den zu behandelnden Gütern auf den Wägeteller 1 aufgesetzt und die Anlage eingeschalten. Das Ablaufdiagramm in Fig. 2 gibt dann den Teil der Gewichtserfassung und Wasserzugabe des Gesamtablaufdiagrammes wieder. Es wird nach Einschalten der Vorrichtung zunächst das Gewicht des mit zu behandelndem Gut gefüllten Behältnisses über die Wägezelle 3 ermittelt, was in Feld 2 unter "Lesen der Waagedaten" angegeben ist. Dabei wird über eine Wägezelle oder eine sonstige elektronische Waage das Gewicht des eingebrachten Gutes erfaßt, da ja das Behältnis als solches ein vorgegebenes Normgewicht aufweist. Eventuelle "falsche Werte" dienen dabei der Erkennung von Fehlersituationen. Wird das Gewicht nun als richtig erkannt, wird die Wassermenge berechnet (siehe Feld 3 "Berechnung der Wassermenge"). Diese Wassermenge wird dann lt. dem in den Fig. 4 und 5 wiedergegebenen Algorithmus errechnet, wobei die für die zuverlässige Desinfektion bzw. Sterilisation erforderliche Wassermenge ermittelt wird. Dabei werden natürlich Erfahrungswerte in den Algorithmus mit eingerechnet. Da sich normalerweise nicht mehr als 10 kg Müll in einem Kübel befinden, wird dieser Wert als Grenzwert angenommen, sodaß jeder Kübel mit über 10 kg Gewicht nicht mehr als 1 Liter bzw. eine Einheit Wasser zugesetzt erhält. Als Mindestmenge Müll hat sich herausgestellt, daß etwa 2 kg ein realistischer Wert ist, d.h. daß dieser Wert etwa 1,5 kg des Kübelgewichtes und 1/2 kg Müll enthält. In diesem Fall soll die Flüssigkeitszugabe 5 Liter (5 Einheiten) betragen. Hat nun der auf den Wägeteller 1 aufgesetzte Behälter bzw. Kübel ein Gesamtgewicht von 4 kg, so ist die Wassermenge, die zuzusetzen ist, dem Algorithmus gemäß Fig. 4 und 5 leicht zu entnehmen; es ist eine Wassermenge von 4 l zuzusetzen. Dies erfolgt dann im nächsten Schritt, welcher im Ablaufdiagramm mit "Flüssigkeitszugabe" angeführt ist. Dabei wird Wasser mit mit den eventuellen Zusätzen aus dem Behälter 8 in das das zu behandelnde Gut enthaltende Behältnis über die Einspritzdüse 5 eingebracht. Ist dieser Schritt beendet, dann beginnt der normale Erhitzungs-, Desinfektions- bzw. Sterilisationszyklus des Gerätes, je nachdem, was eben eingestellt ist. Dies ist im Ablaufdiagramm gemäß Fig. 2 mit "Start des Desinfektionszyklus" angedeutet, nach dessen Beendigung dann das Gerät ausgeschaltet wird.

Wie schon eingangs angedeutet, kann die Wasserzugabe selbstverständlich auch dadurch erfolgen, daß nach Ermitteln des Gewichtes des Kübels die ermittelte Wassermenge bereits außerhalb des Behandlungsraumes 10 in das Behältnis eingebracht ist, sodaß sich der Aufsetzmechanismus für den Düsenkopf 5, also der Stellantrieb 4, erübrigt. Eine solche manuelle Ausführung hat jedoch den Nachteil, daß eventuell erforderliche Wasserzugaben während des Betriebes nicht vorgenommen werden können.

Eine weitere Abwandlung des erfindungsgemäßen Gerätes könnte auch darin gelegen sein, daß die Wägezelle 3 entsprechend dem zu behandelnden Gut voreingestellt werden kann, d.h. daß leichter Müll, wie z.B. zu sterilisierender Zellstoff od.dgl., oder schwerer Müll, wie Küchenabfülle oder Metallgegenstände, bei der Einstellung der Wägezelle bzw. bei der Einstellung des Steuerprogrammes der Mikroprozessorsteuerung 7 berücksichtigt werden können.

Als Desinfektions- und/oder Duftstoffzusätze für die Mikrowellenabfalldesinfektion im vorhin beschriebenen Gerät haben sich Zitronensäure/Zitronenöl als besonders günstig erwiesen, da diese Mittel erst bei höheren Temperaturen die entsprechende Wirkung entfalten, wobei kalte Zitronensäure/kaltes Zitronenöl im normalen Umgang völlig ungefährlich sind. Die Zudosierung der Zitronensäure bzw. des Desinfektions- und/oder Duftmittels kann dabei in Abhängigkeit der über die Mikroprozessorsteuerung zugesetzten Wassermenge erfolgen, d.h., daß in der Mischkammer eine für den Gesamtinhalt des zu behandelnden Gutes vorgegebene absolute Menge eingegeben wird, die dann je nach Menge des zugegebenen Wassers so verdünnt wird, daß innerhalb des zu behandelnden Gutes eine ausreichende Konzentration an Zitronensäure bzw. Desinfektions- und/oder Duftmittel vorliegt. Ist nämlich in dem auf den Wägeteller 1 aufgesetzten Behältnis bereits eine große Flüssigkeitsmenge vorgegeben, dann wird die über die Mischkammer 6 zugesetzte Flüssigkeit eine höhere Konzentration an Desinfektions- und/oder Duftstoff aufweisen, da die zugesetzte Flüssigkeitsmenge ja dann von der bereits im Behälter befindlichen Flüssigkeit verdünnt wird, sodaß im Gesamten gesehen, die erforderliche Konzentration gegeben ist. Ist hingegen das zu behandelnde Gut trocken, dann wird ohnedies eine entsprechende Flüssigkeitsmenge über die Leitung 14 und die Mischkammer 6 der Leitung 13 zugeführt, sodaß dann die in der Leitung 13 befindliche Desinfektionsmittelmenge eben bereits entsprechend verdünnt dem zu behandelnden trockenen Gut zugesetzt ist, sodaß auch hier nur die gerade notwendige Konzentration in dem zu behandelnden Gut vorliegt. Ein weiterer Grund, warum sich Zitronensäure/Zitronenöl als Desinfektions- und/oder Duftstoff anbietet, liegt darin, daß die Abfälle nach der Sterilisation bzw. Desinfektion oder Erhitzen leicht entsorgt werden können, da die Zitronensäure/Zitronenöl die Abwässer nicht schädlich belastet.

Zu der Wassereinbringeinrichtung 5 zu der Mikroprozessorsteuerung 7 führt noch eine Steuerungsleitung 17, welche von der Zugabeeinrichtung 5 wegführt. In dieser Zugabeeinrichtung 5 sind Sensoren 18 für die Erfassung physikalischer und/oder chemischer Parameter angeordnet, wobei insbesondere vorliegend ein Temperaturfühler gemeint ist. Der Temperaturfühler sitzt dabei direkt an der Wandung des in den Behälter reichenden Bereiches der Zugabeeinrichtung 5, wobei die Wandung dieses Bereiches aus mikrowellenundurchlässigem, gut wärmeleitendem Material besteht. Es kann auch in nicht dargestellter Weise der Kopf der Zugabeeinrichtung 5 einen Drucksensor beeinhalten, wobei bei entsprechender Wahl des Wandungsmaterials Bereiche der Wandung des Kopfes oder, wenn erwünscht, der gesamte in das Behältnis ragende Kopfpereich als Drucksensormembrane wirken kann.

In gleicher Weise könnten innerhalb dieser Zugabeeinrichtung, ebenfalls von Hochfrequenzfeldern geschützt, auch andere Sensoren untergebracht werden, und zwar, wie schon eingangs angeführt, gegebenenfalls Sauerstoffelektroden, pH-Elektroden od.dgl.

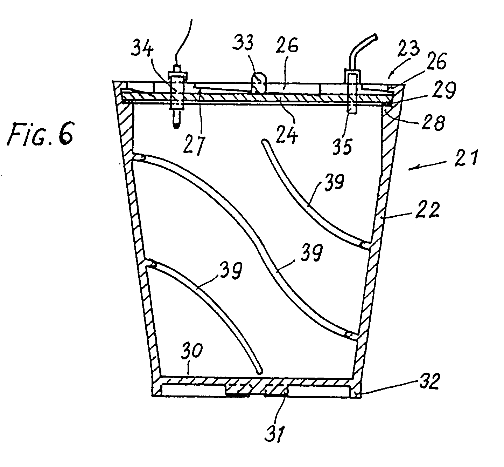

Der mit 21 bezeichnete Behälter weist einen Rumpf 22 mit vorliegende konischer Form auf. Am oberen Rand ist der Behälterrumpf 22 mit einem Bajonettverschluß 23 versehen, der mit an einem Deckel 24 vorgesehenen Gegenstücken 25 zusammenwirkt. Dazu weist der Behälterrumpf 22 am oberen Ende nach innen gerichtete Vorsprünge 26 auf, die an ihren unteren Seiten mit Keilflächen 27 versehen sind (s. Fig. 6). Unterhalb der Vorsprünge 26 ist eine umlaufende Schulter 28 vorgesehen, auf welcher, vorliegend unter Zwischenlage eines Dichtringes 29, der Deckel 24 dicht aufliegt.

Am Boden 30 des Behälterrumpfes 22 sind Mitnehmerrippen 31 vorgesehen, die mit der Oberfläche des im Mikrowellengerät vorgesehenen Drehtellers zusammenwirken. Am Umfang des Bodens 30 sind zudem noch umlaufende Leisten 32 vorgesehen, die den Drehteller außen übergreifen und so ein Herabgleiten des Behälters 21 oder ein Verschieben desselben am Drehteller verhindern. Es könnte in gleicher Weise auch eine entsprechende Vertiefung oder Einziehung der Unterseite des Bodens vorgesehen sein.

Am Deckel 24 ist ein Griff 33 vorgesehen, mittels welches der Deckel 24 nach Einführen der Gegenstücke 25 zwischen die Vorsprünge 26 verdreht werden kann, damit die Gegenstücke 25 unter die Vorsprünge 26 gedreht und entlang der Keilflächen 27 schräg nach unten bewegt werden können, wodurch der Deckel 24 mit seiner Unterseite gegen den Dichtring 29 gepreßt wird. Weiters sind am Deckel 24 noch Anschlüsse und Armaturen vorgesehen, wie beispielsweise ein Thermofühler 34. Entlang der Innenwandung sind verlaufende Mischleisten 39 angeordnet, mittels welcher im Behälter befindliche Flüssigkeiten oder im Behälter befindliches breiiges Gut bei Drehung des Behälters 1 gemischt werden können. Vorliegend sind gleichsinnig schraubenlinig verlaufende Mischleisten dargestellt, jedoch könnten diese Mischleisten auch gegenläufig, bevorzugt abwechselnd, angeordnet sein. Die Enden und auch die nach innen weisenden Kanten der Mischleisten 19 sind abgerundet, damit bei Verwendung des Behälters 1 mit eingesetztem Kunststoffsack eine Zerstörung dieses Sackes ebenfalls verhindert ist.

Als Material für den erfindungsgemäßen Behälter wird ein für Mikrowellen durchlässiger Kunststoff eingesetzt, der temperaturbeständig und druckfest ist. Dazu eignet sich besonders Teflon, jedoch können auch alle anderen Materialien eingesetzt werden, die den angeführten Anforderungen entsprechen.

Anhand der Zeichnungen sind solche Ausführungsformen beschriebenworden, bei welchen der Deckel von oben in die Behälteröffnung eingesetzt wird. In nicht dargestellter Weise könnte auch eine Ausführung gewählt werden, bei welcher der Deckel einen den Bellälterrand außen übergreifenden Rand hat, wobei die Vorsprünge am Behälter dann nach außen gerichtet sind und mit nach innen gerichteten Gegenstücken am Deckel zusammenwirken.

## Patentansprüche

1. Verfahren zum Erhitzen, Desinfizieren oder Sterilisieren von Gütern, in welcher das zu behandelnde Gut mittels Mikrowellen erhitzt wird, wobei in die das zu behandelnde Gut enthaltende Behandlungskammer Flüssigkeit, z.B. Wasser, welche gegebenenfalls mit Zusätzen, z.B. mit Desinfektionsmittel und/oder Duftstoffen, versetzt ist, eingebracht wird, dadurch gekennzeichnet, daß die zuzugebende Flüssigkeitsmenge in Abhängigkeit vom Wassergehalt des zu behandelnden Gutes bestimmt und direkt diesem zugesetzt wird.

2. Anlage zum Erhitzen, Desinfizieren oder Sterilisieren von Gütern, in welcher das zu behandelnde Gut mittels Mikrowellen erhitzt wird, zur Durchführung des Verfahrens nach Anspruch 1, wobei in die das zu behandelnde Gut enthaltende Behandlungskammer Flüssigkeit, z.B. Wasser, welche gegebenenfalls mit Zusätzen, z.B. mit Desinfektionsmittel und/oder Duftstoffen versetzt ist, einbringbar ist, dadurch gekennzeichnet, daß für die Ermittlung des Wassergehaltes des zu behandelnden Gutes eine auf das Gewicht desselben ansprechende Meßeinrichtung (3) vorgesehen ist.

3. Anlage nach Anspruch 2, dadurch gekennzeichnet, daß die Meßeinrichtung (3) als Wägezelle ausgebildet ist, welche mit einer Steuereinrichtung (7) für die Flüssigkeitszugabe verbunden ist.

4. Anlage nach Anspruch 3, dadurch gekennzeichnet, daß die Steuereinrichtung der Flüssigkeitszugabe anhand eines den tatsächlichen Flüssigkeitsgehalt berücksichtigenden Algorithmus steuerbar ist (Fig. 4, 5).

5. Anlage nach Anspruch 3 oder 4, dadurch gekennzeichnet, daß die Wägezelle (3) außerhalb des Resonanzraumes (10) der Mikrowellen angeordnet ist und als Auflager für die den Behälter (21) für das zu behandelnde Gut bildende tragende Platte (1) bzw. Wandung dient.

6. Anlage nach einem der Ansprüche 3 bis 5, dadurch gekennzeichnet, daß die Flüssigkeitszugabeeinrichtung (5) zur Zuführung der Flüssigkeit in das zu behandelnde Gut innerhalb der Behandlungskammer (10) angeordnet ist.

7. Anlage nach Anspruch 6, dadurch gekennzeichnet, daß die aus mikrowellenundurchlässigem Material gefertigte Zugabeeinrichtung (5) in eine Öffnung der Behandlungskammer (10) einsetzbar ist, wobei in dem in das Innere der Behandlungskammer ragenden Kopf der Zugabeeinrichtung (5) Sensoren (18) für die Erfassung physikalischer und/oder chemischer Parameter angeordnet sind.

8. Anlage nach Anspruch 7, dadurch gekennzeichnet, daß der Kopf der Zugabeeinrichtung (5) selbst als Temperaturfühler ausgebildet ist.

9. Anlage nach Anspruch 7 oder 8, dadurch gekennzeichnet, daß der Kopf der Zugabeeinrichtung (5) als Druckfühler ausgebildet ist, wobei vorzugsweise die Wandung bzw. Bereiche davon als Druckmembrane ausgebildet ist.

10. Anlage nach einem der Ansprüche 2 bis 9, dadurch gekennzeichnet, daß der Kopf der Zugabeeinrichtung (5) nach Schließen der Behandlungskammer (10) auf den Behälter (21) absenkbar ist, welcher in seinem Deckel mit einer vom Kopf der Zugabeeinrichtung (5) abschließbaren Öffnung versehen ist.

11. Anlage nach einem der Ansprüche 2 bis 4 und 6 bis 10, dadurch gekennzeichnet, daß zur Auflage des Behälters (21) ein Drehteller vorgesehen ist, wobei an der Außenseite des Bodens (30) des Behälters (21) Mitnahmeeinrichtungen (31) für das Zusammenwirken mit dem Drehteller angeordnet sind.

12. Anlage nach Anspruch 11, dadurch gekennzeichnet, daß an der Außenseite des Bodens (20) des Behälters (21) zusätzlich oder anstelle der Mitnahmeeinrichtungen (31) Halteeinrichtungen (32) gegen ein Abgleiten des Behälters vom Drehteller vorgesehen sind.

13. Anlage nach Anspruch 11 oder 12, dadurch gekennzeichnet, daß an der Innenseite der Wandung des Behältes (22) nach innen vorspringende, vorzugsweise mit abgerundeten Enden und/oder Kanten versehene Mischleisten (39) od.dgl. angebracht sind.

14. Anlage nach Anspruch 13, dadurch gekennzeichnet, daß die Mischleisten (39) schraubenlinienförmig verlaufen, wobei die Mischleisten durchgehend oder als unterbrochene Abschnitte ausgebildet sind.

15. Anlage nach Anspruch 13 oder 14, dadurch gekennzeichnet, daß die Mischleisten (39) alle gleichsinnig oder, vorzugsweise abwechselnd, gegenläufig angeordnet sind.

## Claims

1. Method of heating, disinfecting or sterilising goods in which the goods to be treated are heated by means of microwaves, whereby liquid, e.g. water, which is optionally mixed with additives, e.g. with disinfectant and/or fragrances, is introduced into the treatment chamber containing the goods to be treated, characterised in that the amount of liquid to be added is determined in dependence on the water content of the goods to be treated and is added directly to them.

2. Installation for heating, disinfecting or sterilising goods, in which the goods to be treated are heated by means of microwaves, for carrying out the method as claimed in Claim 1, whereby liquid, e.g. water, which is optionally mixed with additives, e.g. with disinfectant and/or fragrances, may be introduced into the treatment chamber containing the goods to be treated, characterised in that for the purpose of determining the water content of the goods to be treated a measuring device (3) responsive to the weight of the same is provided.

3. Installation as claimed in Claim 2, characterised in that the measuring device (3) is constructed as a weighing cell which is connected to a control device (7) for the addition of liquid.

4. Installation as claimed in Claim 3, characterised in that the control device for the addition of liquid is controllable with reference to an algorithm which takes account of the actual liquid content (Figs. 4, 5).

5. Installation as claimed in Claim 3 or 4, characterised in that the weighing cell (3) is arranged outside the resonance space (10) of the microwaves and serves as a support for the plate (1) or wall which supports the container (21) for the goods to be treated.

6. Installation as claimed in one of Claims 3 to 5, characterised in that the liquid addition device (5) for supplying the liquid into the goods to be treated is arranged within the treatment chamber (10).

7. Installation as claimed in Claim 6, characterised in that the addition device, which is manufactured from microwave-impermeable material, is insertable into an opening in the treatment chamber (10), sensors (18) for determining physical and/or chemical parameters being arranged in the head of the addition device (10) which projects into the interior of the treatment chamber.

8. Installation as claimed in Claim 7, characterised in that the head of the addition device (5) is itself constructed as a temperature sensor.

9. Installation as claimed in Claim 7 or 8, characterised in that the head of the addition device (5) is constructed as a pressure sensor, the wall or regions thereof being preferably constructed as a pressure membrane.

10. Installation as claimed in one of Claims 2 to 9, characterised in that the head of the addition device (5) is lowerable, after closing the treatment chamber (10), onto the container (21) which is provided in its lid with an opening which may be closed by the head of the addition device (5).

11. Installation as claimed in one of Claims 2 to 4 and 6 to 10, characterised in that a rotary plate is provided to support the container (21), carriers (31) for cooperating with the rotary plate being arranged on the outer side of the base (30) of the container (21).

12. Installation as claimed in Claim 11, characterised in that provided on the outer side of the base (20) of the container (21) in addition to or instead of the carriers (31) there are retaining devices (32) against the container slipping off the rotary plate.

13. Installation as claimed in Claim 11 or 12, characterised in that inwardly projecting mixing bars (39) or the like, preferably with rounded ends and/or edges, are attached to the inner side of the wall of the container (22).

14. Installation as claimed in Claim 13, characterised in that the mixing bars (39) extend helically and are continuous or constructed as interrupted sections.

15. Installation as claimed in Claim 13 or 14, characterised in that the mixing bars (39) are all arranged in the same sense or, preferably alternately, in the opposite sense.

## Revendications

1. Procédé de chauffage, désinfection ou stérilisation de produits, dans lequel le produit à traiter est chauffé par des micro-ondes, un liquide, par exemple de l'eau, qui est mélangée, le cas échéant, avec des additifs, par exemple avec des produits désinfectants et/ou avec des matières odoriférantes, étant placé dans la chambre de traitement contenant le produit à traiter, caractérisé en ce que la quantité de liquide à ajouter est déterminée par la teneur en eau du produit à traiter et lui est directement ajoutée.

2. Installation destinée à chauffer, désinfecter ou stériliser des produits, dans laquelle le produit à traiter est chauffé par des micro-ondes, destinée à mettre en oeuvre le procédé selon la revendication 1, un liquide, par exemple de l'eau, qui est mélangée, le cas échéant, avec des additifs, par exemple avec des produits désinfectants et/ou avec des matières odoriférantes, pouvant être placé dans la chambre de traitement contenant le produit à traiter, caractérisée en ce qu'est prévu, pour déterminer la teneur en eau du produit à traiter, un dispositif de mesure (3) sensible au poids de celui-ci.

3. Installation selon la revendication 2, caractérisée en ce que le dispositif de mesure (3) est conformé en cellule de pesée, qui est reliée à un dispositif de commande (7) pour l'ajout de liquide.

4. Installation selon la revendication 3, caractérisée en ce que le dispositif de commande pour l'ajout de liquide est susceptible d'être commandé par un algorithme tenant compte de la teneur réelle en liquide (fig. 4, 5).

5. Installation selon la revendication 3 ou 4, caractérisée en ce que la cellule de pesée (3) est placée en-dehors de la cavité résonnante (10) des micro-ondes, et sert d'appui pour la plaque (1) et/ou la paroi portant le récipient (21) destiné au produit à traiter.

6. Installation selon l'une des revendications 3 à 5, caractérisée en ce que le dispositif (5) pour l'ajout de liquide est disposé pour amener le liquide dans le produit à traiter à l'intérieur de la chambre de traitement (10).

7. Installation selon la revendication 6, caractérisée en ce que le dispositif d'ajout (5) en matériau laissant passer les micro-ondes est susceptible d'être placé dans une ouverture de la chambre de traitement (10), des capteurs (18) pour la détection de paramètres physiques et/ou chimiques étant placés dans la tête du dispositif d'ajout (5) qui pénètre à l'intérieur de la chambre de traitement.

8. Installation selon la revendication 7, caractérisée en ce que la tête du dispositif d'ajout (5) est elle-même conformée en capteur de température.

9. Installation selon la revendication 7 ou 8, caractérisée en ce que la tête du dispositif d'ajout (5) est conformée en capteur de pression, la paroi et/ou des zones de celle-ci étant conformée(s), de préférence, en membrane de pression.

10. Installation selon l'une des revendications 2 à 9, caractérisée en ce que la tête du dispositif d'ajout (5), après la fermeture de la chambre de traitement (10), est susceptible d'être abaissée sur le récipient (21), dont le couvercle est pourvu d'une ouverture susceptible d'être obturée par la tête du dispositif d'ajout (5).

11. Installation selon l'une des revendications 2 à 4 et 6 à 10, caractérisée en ce qu'est prévu, pour le support du récipient (21), un plateau tournant, des dispositifs d'entraînement (31) étant prévus, sur la face extérieure du fond (30) du récipient (21), pour coopérer avec le plateau tournant.

12. Installation selon la revendication 11, caractérisée en ce que sont prévus, sur la face extérieure du fond (30) du récipient (21), en supplément ou à la place des dispositifs d'entraînement (31), des dispositifs de maintien (32) empêchant le récipient de glisser du plateau tournant.

13. Installation selon la revendication 11 ou 12, caractérisée en ce que sont placées, sur la face intérieure de la paroi du récipient (22), des nervures mélangeuses (39) dépassant vers l'intérieur, présentant de préférence des extrémités et/ou arêtes arrondies, ou similaires.

14. Installation selon la revendication 13, caractérisée en ce que les nervures mélangeuses (39) sont placées sur une ligne hélicoïdale, les nervures mélangeuses étant continues ou placées en tronçons interrompus.

15. Installation selon la revendication 13 ou 14, caractérisée en ce que les nervures mélangeuses (39) sont orientées dans le même sens ou sont placées, de préférence, de façon alternative en sens opposé.
